# EUROPEAN PATENT APPLICATION

(11) **EP 4 385 555 A1**
(43) Date of publication of application: **19.06.2024**
(21) Application number: 22213591.5
(22) Date of filing: 14.12.2022
(51) Int. Cl.: A61M 39/28

(54) **MULTIWAY PINCH DEVICE FOR SELECTIVE FLEXIBLE TUBES PINCHING**

(71) Applicant: SARTORIUS STEDIM FMT SAS, 13400 Aubagne (FR)
(72) Inventor: Deuse, Mario, 37120 Bovenden (DE); Weisshaar, Stefan, 37139 Adelebsen (DE)
(74) Representative: Derambure Conseil

(57) **Abstract**

A device for holding a set of M flexible tubes and for selectively pinching or releasing all or a subset of N tubes, the device comprising a body (1), having M passages (12) configured to accommodate one tube, a set of pinch elements (4), each pinch element guided in a guide slot and radially movable in the passage with regard to the main axis A, each pinch element being configured to selectively pinch or release one tube, a pivotal selector hub (2), wherein an angular position of the selector hub around the main axis causes to select the subset of N tubes, by selecting a corresponding subset of pinch elements to be allowed to move, a pivotal control member (3) having a cam portion (32) configured to cause or allow a radially oriented movement of pinch elements when the control member is rotated.

## Description

### FIELD OF THE DISCLOSURE

The present invention relates to a flow distribution device. More precisely, the disclosure relates to multiway pinch device to be used to control a bio pharmaceutical process and generally bioprocess systems. The present disclosure is of particular relevance for arrangements using single-use components.

### BACKGROUND OF THE DISCLOSURE

We know pinch valves for fluid delivery devices for example used on tubes in single-use liquid handling systems used in the bioprocess domain.

Single-use systems employ typically sterilized components that are in fluid contact with the processed fluid of interest. Pinch valves are often used with such disposable single-use components due to the simple and cost-effective principle.

Regarding a pinch valve, there is provided at least a flexible portion of the tube, the pinch valve acting on this flexible portion, on the outer wall thereof. There is provided at least a pinch element also called clamping element. The valve is in a "closed" state when the pinch element squeezes the flexible tube and obstruct any fluid flow within the tube. The valve is in a "open" state when the tube is not squeezed, i.e., not pinched, and due to its intrinsic resilience, the tube comes to a rest shape where fluid is allowed to flow along the tube. There is no interaction between the pinch valve and the fluid itself, thereby contributing to preserving sterile conditions of the fluid.

WO2020126638 promotes a manifold controlled by a plurality of pinch valves. However, this arrangement requires an individual control of each pinch valve, and therefore is complex and not cost-effective.

The inventors have sought to propose a cost-effective solution to control a plurality of pinch valves with a single optimized device.

### SUMMARY OF THE DISCLOSURE

There is therefore proposed a device (distribution device) for holding a set of **M** flexible tubes and for selectively pinching or releasing all or a subset of **N** tubes among the set of **M** flexible tubes, the device comprising:

- a body **(1),** having at least **M** passages **(12),** each passage being configured to accommodate one tube, the body having a main axis (A),

- a set of pinch elements **(4),** each pinch element being guided in a guide slot and radially movable in the passage about the main axis **A,** each pinch element being configured to selectively pinch or release one tube,

- a selector hub **(2),** pivotally mounted within the body about the main axis **A,** wherein an angular position of the selector hub around the main axis causes to select the subset of **N** tubes, by selecting a corresponding subset of pinch elements to be allowed to move,

- a control member **(3)** pivotally mounted within the body about the main axis, the control member having a cam portion **(32)** configured to cause or allow a radially oriented (outwards or inwards) movement of some the pinch elements when the control member is rotated around the main axis,

- a mechanical coupling arrangement **(24,34),** wherein the control member is coupled in rotation with the selector hub for allowing a free relative rotation along a first angular range and a rotationally rigid engagement beyond said first angular range.

Thanks to this arrangement, a rotation of the selector hub causes the selector hub to select the proper tube(s) to act upon, i.e., change valve state, and a rotation of the control member in either direction causes to respectively and selectively pinch or release the considered tube(s).

More precisely, the rotation of the selector hub causes the selector hub to select a corresponding subset of pinch elements to be allowed to move, corresponding to the subset of tubes.

In one particular non-limiting example, only one pinch element and one corresponding tube is allowed to change state, the selector hub maintaining the other tubes in the current state (pinched or released). In one particular non-limiting example, the selector hub maintains the other tubes in the current pinched state, only one tube is allowed to be released by a corresponding pinch element.

Generally, the number **M** of passages is at least 2, often more than 3 or 4.

It should be appreciated there is provided for each tube a valve function, wherein the valve is in a "open" state when the tube is not squeezed, and due to its intrinsic resilience, comes to a rest shape where fluid is allowed to flow within the tube. It should further be appreciated that at least a portion of the tube located in the valve area is flexible, other portions of the tube may be rigid. The valve is in a "closed" state when the tube is squeezed.

The promoted distribution device is a multi-way distribution device, i.e. a M-way distribution device. As stated in detail later, the promoted distribution device prevents any cross contamination between the different ways of the multi-way distribution device.

In various embodiments of the invention, one may possibly have recourse in addition to one and/or other of the following arrangements, taken alone or in combination.

According to a preferred option, the cam portion **(32)** is configured to cause a radially outwards movement of some of the pinch elements when the control member is rotated around the main axis, whereas in normal condition the tube is not pushed by the pinch element. The intrinsic resilience the tubes biases the pinch elements radially inwards when the cam portion does not push outwardly the pinch element.

According to an alternate option, the cam portion is configured to cause a radially inwards movement of some pinch elements when the control member is rotated around the main axis. In this case, there is provided a biasing system to push the pinch elements radially outwards to return to the rest position.

According to an option, a number **M-N** tubes are normally pinched by the pinch elements and the selector hub, and the device is configured to release only one or two selectable tubes, i.e., N =1 or N=2. In practice, according to examples, **M** is chosen within an interval **[4-12],** preferably within an interval **[6-10].**

According to one aspect, for the **M-N** normally pinched tubes, the pinch elements are pushed outwardly at least by the selector hub. For the one or two selectably releasable tubes, the pinch elements are pushed outwardly or let move inwardly by the control member.

According to an option, the selector hub **(2)** comprises a latch member **(58)** configured to prevent a rotation of the selector hub along a first rotational direction [e.g., clockwise] and to allow a rotation of the selector hub along a second rotational direction (e.g., counterclockwise). In other words, the latch member provides a one-way rotational movement of the selector within the body. In addition, when the control member is rotated along the first rotational direction (e.g., clockwise) the cam portion **(32)** causes a radial movement of some the pinch elements. i.e., let it move inwards

The latch member can be a pawl or an elastic finger.

According to an option, the latch member **(58)** abuts on shoulders in the body. Embodiments incorporating this option are believed to be easy to implement together with the latch member.

According to an option, the control member comprises a top protrusion that can be manually actuated, i.e., by fingers of a user. Embodiments incorporating this option is believed to have a simple design, which is cost effective and intuitive.

According to an option, the top protrusion indicates, by its direction or an arrow thereon, the tube position selected for releasing the pinch action, i.e., opening the pinch valve. Embodiments incorporating this option are believed to provide an intuitive use of the distribution valve.

According to an option, the control member is rotated via an output shaft controlled by a motorized actuator. We therefore provide a motor driven valve, with possibility to actuate the distribution device remotely.

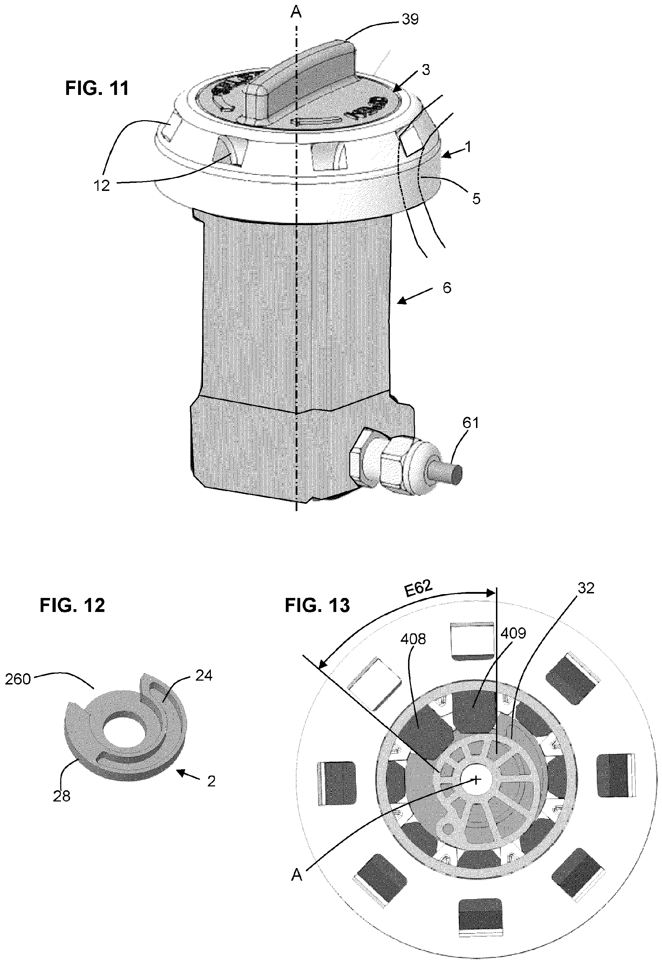

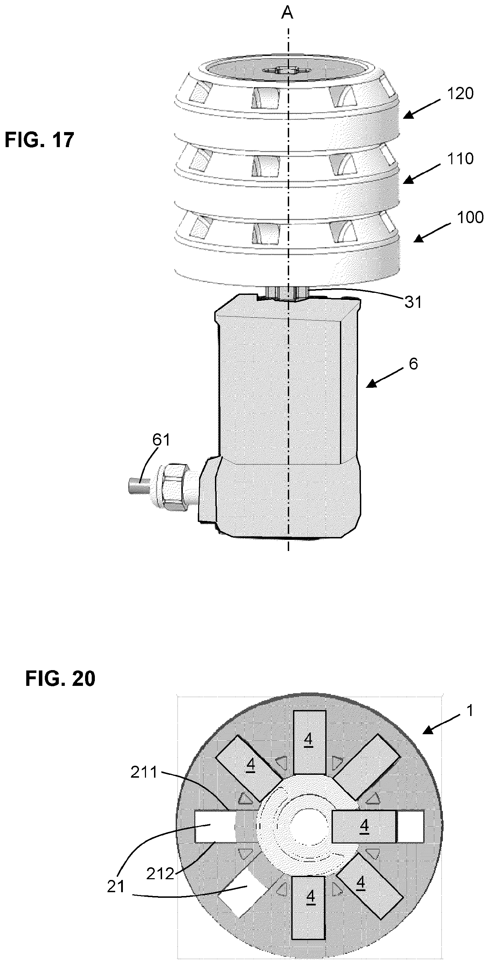

The present disclosure therefore is also directed to an assembly comprising the distribution device described above, a motorized actuator having an output shaft coupled to the distribution device. **(See** **FIG. 11** **and** **17****).**

According to an option, the first angular range is comprised between 100° and 180°. Embodiments incorporating this option are believed to enable a smooth cam actuation, the cam ramp is not much inclined.

According to an option, the mechanical coupling arrangement is such that the control member comprises a pin **(34)** engaged in an arcuate groove **(24)** arranged in the selector hub, the available angular stroke of the pin within the arcuate groove defining the first angular range. Embodiments incorporating this option are believed to forms a simple and reliable solution to provide free relative rotation along the first angular range and a rotative driving function beyond said first angular range.

According to an option, the passages are disposed in a circle and passages are regularly spaced circumferentially. Embodiments incorporating this option are believed to enable to optimize the occupied space.

It is understood that the arrangement along the circumferential direction can be different from a regularly spaced arrangement, e.g. with unequal angular spacing, or with only a sector < 360° carrying the passages.

According to an option, **M** is at least 4 and the device has at least 4 passages and accommodate at least 4 tubes. Embodiments incorporating this option are believed to accommodate a large variety of systems which can use such a distribution device.

According to an option, **N** is equal to 1. Embodiments incorporating this option are believed to provide that all the tubes are pinched but one, and any tube can be chosen to be released, thereby exhibiting maximum flexibility and selectivity.

According to an option, the selector hub can comprise an outer rim **(28)** interrupted by a recess **(26).** Embodiments incorporating this option are believed to cause the recess allows one or two pinch elements to move inwardly.

According to an option, each passage comprises an insertion slot **(95).** Embodiments incorporating this option are believed to allow inserting the tubes very easily in the passage along the radial inwards direction. It should be appreciated that thanks to the flexibility of the tube, the insertion slot is much smaller than the outer diameter of the tube at rest. Accordingly, it is not necessary to insert the tube from the bottom of the passage or from the top of the passage. Thus, in some embodiments, it is therefore possible to insert an already connected tube into the passage via the slot even though the tube is continuously connected to other devices on both ends.

According to an option, there is provided sensing means **(7)** to sense the radial position of each pinch element.

According to an option, each pinch element may comprise a magnet **(71)** and there is provided a Hall-effect sensor **(72)** located opposite the magnet when pinch element pushed radially outwards, i.e., when the tube is pinched. This allows to control proper operation of the pinch elements and closed-loop control in a motorized configuration.

According to an option, Hall-effect sensors are mounted on a printed circuit board, i.e., a PCB.

According to an option, there is provided biasing members **(9)** to push the pinch elements radially inwards. This complements the tube resilience.

According to an option, the front portion of each pinch element is a wedge-shape.

The present disclosure also relates to an assembly comprising at least two devices as described above and a control shaft controlled by motorized actuator.

According to a second aspect of the present disclosure, which can be considered independently from the first aspect above, there is proposed a device for holding a set of **M** flexible tubes and selectively pinching or releasing all the set of M flexible tubes, the device comprising:
- a body **(1),** having **M** passages **(12),** each passage being configured to accommodate one tube, the body having an axis **A,**
- a set of pinch elements **(4),** each pinch element being guided in a guide slot and radially movable in the passage about the axis **A,** each pinch element being configured to selectively pinch or release one tube,
- a control member **(3)** pivotally mounted within the body about the axis A, with a mechanical interaction between the control member and the pinch elements to cause a radially oriented movement of all the pinch elements altogether when the control member is rotated around the axis.

According to an option, the device as described above, wherein the mechanical interaction is formed by a set of connecting rods **(8),** wherein each connecting rod has a first end **(81)** coupled to a back end of one pinch element, and a second end **(82)** coupled to an attachment portion of the control member.

According to an option, the mechanical interaction is formed by one or more cam portion provided on the control member and a corresponding cam follower area on each back end of respective pinch elements.

According to an option, there is provided a predefined angular range for the control member within the body, with a first angular stop and a second angular stop, wherein when the control member abuts against the first angular stop, the pinch elements are completely withdrawn from the passages, and wherein when the control member abuts against the second angular stop, the pinch elements are caused to pinch the tubes held in the passages to obstruct any fluid flow in the tubes.

According to an option, the predefined angular range available between the first angular stop and the second angular stop is comprised between 25° and 50°.

According to an option, for each connecting rod, the first end is pivotally coupled to the back end of one pinch element with a first joint axis (**X1**), and the second end is pivotally coupled to the attachment portion of the control member with a second joint axis (X2).

According to an option, when the control member abuts against the second angular stop, the main axis **A,** the first joint axis and the second joint axis are substantially aligned for each couple of rod and pinch element.

According to an option, for each connecting rod, there is provided a first pivot pin at the pinch element and a second pivot pin at the control member **(3).**

According to an option, each passage comprises an insertion slot **(95).**

According to an option, **M** is at least 4 and the device has at least 4 passages and accommodate at least 4 tubes.

According to an option, the control member is rotated via an output shaft controlled by a motorized actuator **(6).**

### BRIEF DESCRIPTION OF DRAWINGS

Other features and advantages of the invention appear from the following detailed description of two of its embodiments, given by way of non-limiting example, and with reference to the accompanying drawings.
**FIG. 1A** exemplarily illustrates a diagrammatic view of a bioprocess system involving a device according to an embodiment of the present invention.
**FIG. 1B** exemplarily illustrates a diagrammatic view of another type of system involving a device according to an embodiment of the present disclosure.
**FIG. 2A** exemplarily illustrates a tube within the passage the tube being released.
**FIG. 2B** exemplarily illustrates a tube within the passage the tube being pinched.
**FIG. 3** illustrates an exploded perspective view of a distribution device according to the present disclosure.
**FIG. 4** illustrates a more detailed view of the control member.
**FIG. 5** illustrates a more detailed view of the selector hub.
**FIG. 6** illustrates a more detailed view of the selector hub.
**FIG. 7** shows a bottom view of the selector hub and the control member.
**FIG. 8** shows a section view of the selector hub and the control member
**FIG. 9** shows a perspective section view of the device, according to a variant embodiment, with biasing tongues.
**FIG. 10** illustrates a more detailed view of the biasing tongue.
**FIG. 11** illustrates a further embodiment, in a motorized configuration.
**FIG. 12** and **13** illustrate another embodiment, in which 2 tubes can be selected at the same time. **FIG. 12** illustrates a related selector hub. **FIG. 13** illustrates a related transverse section view.
**FIG. 14** and **15** illustrate still another embodiment, with pinch elements position sensing means. **FIG. 14** illustrates a related exploded perspective view. **FIG. 15** illustrates a related axial section view.

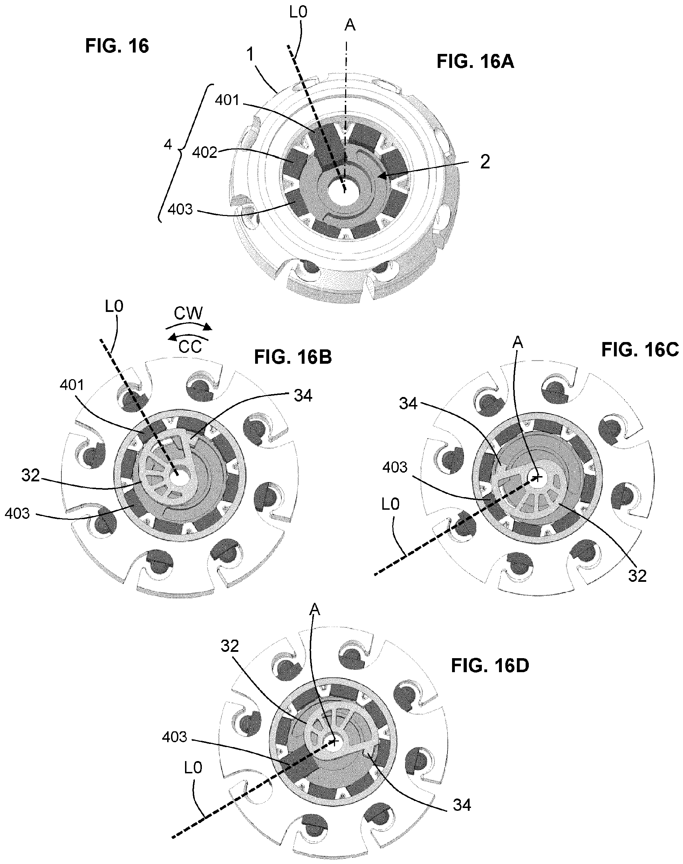

**FIG. 16** illustrates schematically a behavior of the distribution device with different positions, viewed from a top viewpoint. **FIG. 16A** illustrates a first position with a first pinch element retracted. **FIG. 16B** illustrates a second state with the control element rotated in counterclockwise direction and the selector hub still not rotated. **FIG. 16C** illustrates a third state with the control element and the selector hub rotated in counterclockwise direction by an angular range corresponding to two pinch element angular pitch. **FIG. 16D** illustrates a fourth state with the control element rotated in clockwise direction and the selector hub not rotated, thereby allowing a retraction of another pinch element.
**FIG. 17** illustrates a further embodiment with several distribution devices stacked one upon another, together with motorized actuator.
**FIG. 18** and **19** illustrate still another embodiment, with a common control of pinch elements. **FIG. 18A** illustrates close position whereas **FIG. 18B** illustrates open positions. **FIG. 19** illustrates a partial view around a connecting rod.
**FIG. 20** illustrates in further details guide slots receiving the pinch elements. **FIG. 20** is a section view taken at a section line XX-XX shown at Figure 9. Further in FIG. 20 two pinch elements are omitted to enhance clarity.

### DETAILED DESCRIPTION

In the figures, the same references denote identical or similar elements.

In manufacturing of biopharmaceuticals, single-use systems have emerged and are well adapted for liquid handling in bio pharmaceutical preparation systems. Such single-use systems use single-use components that are preferably made from incinerable plastics materials and are often disposed of after use to avoid cleaning prior to re-uses and related cleaning validation. With such single-use systems, we therefore avoid using costly sterilization methods. By having the disposable components sterilized and clean-room manufacturing, all cleaning and cleaning validation prior to processing is also eliminated.

Here we focus on particular on systems using flexible tubes.

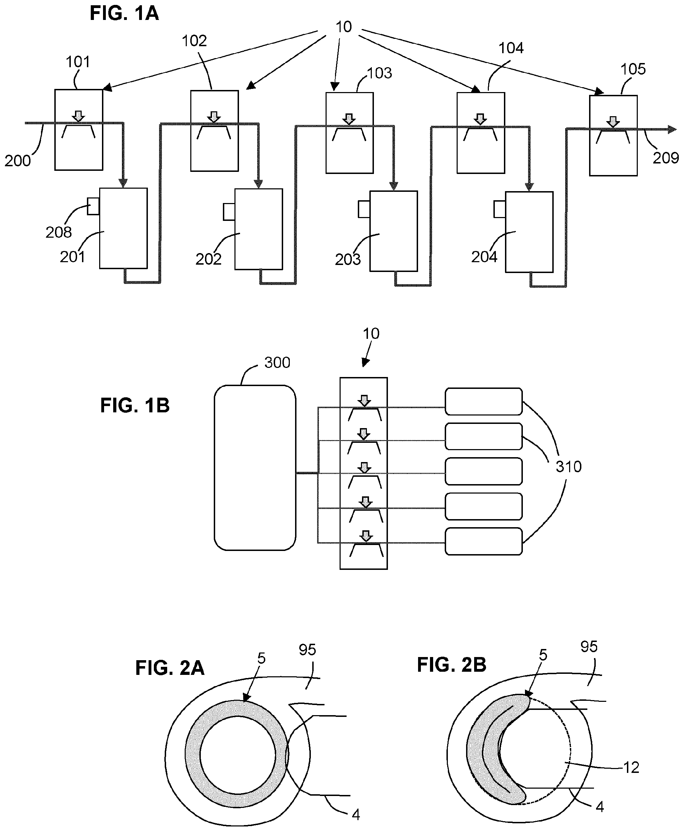

On **FIG. 1A****,** we schematically present an exemplary bioprocess system involving a distribution device **10.** An input channel **200,** for example a flexible tube runs form an input port to a first bioreactor **201** via a first portion **101** of the distribution device **10.** The first portion **101** includes a pinch valve. In the input channel **200** flows any pharmaceutical product of interest. In the first bioreactor **201,** a reactive product can be added from an auxiliary vessel **208.** At the output of the first bioreactor **201,** the resulting pharmaceutical product is directed to a second bioreactor **202** via a second portion **102** of the distribution device **10.** The second portion **102** includes a pinch valve. In the second bioreactor **202,** a reactive product can be added from an auxiliary vessel.

At the output of the second bioreactor **202,** the resulting pharmaceutical product is directed to a third bioreactor **203** via a third portion **103** of the distribution device **10.** In the third bioreactor **203,** a reactive product can be added from an auxiliary vessel.

At the output of the third bioreactor **203,** the resulting pharmaceutical product is directed to a fourth bioreactor **204** via a fourth portion **104** of the distribution device 10. In the fourth bioreactor 204, a reactive product can be added from an auxiliary vessel.

At the output of the fourth bioreactor 204, the resulting pharmaceutical product is directed to an output port **209** via a fifth portion **105** of the distribution device **10.**

The bioreactors **201-204** can be for example made of plastics. The bioreactors **201-204** can be single-use equipment. The various portions **101-105,** e.g., as individual pinch valves, discussed above are arranged in a single unit called therein "distribution device" **10** as it will be discussed below.

Even though the individual pinch valves are arranged in single-unit distribution device, it should be noticed that each individual valves are physically independent from one another. Therefore, there is no possible cross-contamination between the pinch valves, no possible cross-contamination between the flexible tubes, and a fortiori no possible cross-contamination between the pharmaceutical product flowing into those tubes.

Of course, the number of bioreactors arranged in series can be more important, or alternately less.

It is important to note that the in the above arrangement, the successive portions **101-105** of the distribution device **10** are open sequentially one after the other. Each portion can be formed as a pinch valve. In such case, each portion is selectively open to let pharmaceutical product goes through whereas the other ones remain closed. Each bioreactor is activated after its predecessor and before its successor. In such system, there may be provided pumping means, not shown. We note that the system may comprise pumping means, not shown.

On **FIG. 1B****,** another configuration is presented where the distribution device according to the present disclosure can be used. In this configuration, depicted schematically, a large vessel **300** contains a certain amount of pharmaceutical product which is to be distributed across smaller containers/vessels **310.** The number and volume of containers/vessels is not limited per se. It can go down to individual vaccine doses.

In this case, the distribution device comprises one or more parallel channels, each controlled by the pinch valve. The pinch valves can be activated altogether, in a simultaneous manner, or alternatively each pinch valve can be activated selectively.

In the following, the number **M** denotes the number of channels, which is also the number of used passages in the distribution device **10.** In some applications, some of the available passages may remain devoid of any tube.

**FIG. 2A** exemplarily illustrates in cross section a flexible tube **5** within a passage. In this case the pinch element **4** is not interfering with the tube **5.** A flow of a product within the tube is possible. Said otherwise, the passage the tube is released. The state of the pinch valve is also called "open".

**FIG. 2B** by contrast exemplarily illustrates in cross section a pinched tube within the passage **12.** In this case, the pinch element **4** is interfering with the tube **5.** The pinch element **4** presses radially the tube. There is no cross section left to let liquid flow of along the tube **5** at this location. The pinch element **4** is selectively movable radially inwards or outwards. The front end of the pinch element is preferably smooth and rounded to provide intimate contact with the collapsed tube. In some embodiments the front end of the pinch element can exhibit a shape of an arc of circle. The state of the pinch valve is also called "closed". Said otherwise, the flow of liquid in the tube is obstructed.

The slot **95** allows insertion of the flexible into the passage and allows removal of the flexible from the passage after use. Since the slot has a small width, once the flexible tube is located within the passage, it cannot be removed unless an intentional squeeze is practiced. We further note that the pinch element **4,** when pressed against the flexible tube, tends to move the tube away from the slot **95.** The risk of inadvertent escape of the tube from the passage **12** is therefore mitigated or even eliminated.

In absence of interaction of the pinch element, the intrinsic resilience of the flexible tube biases the tube to the circular rest configuration, occupying most of the available cross section area of passage **12.**

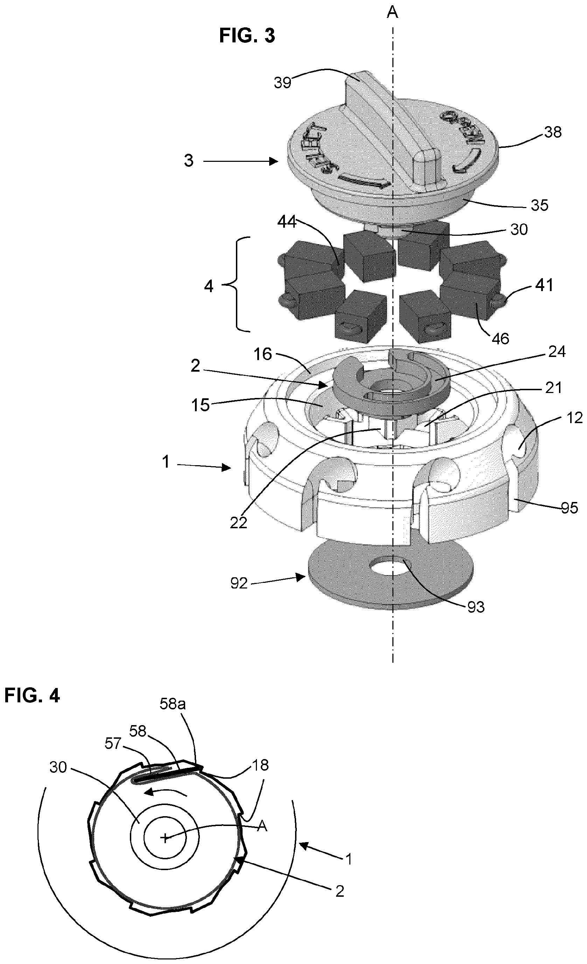

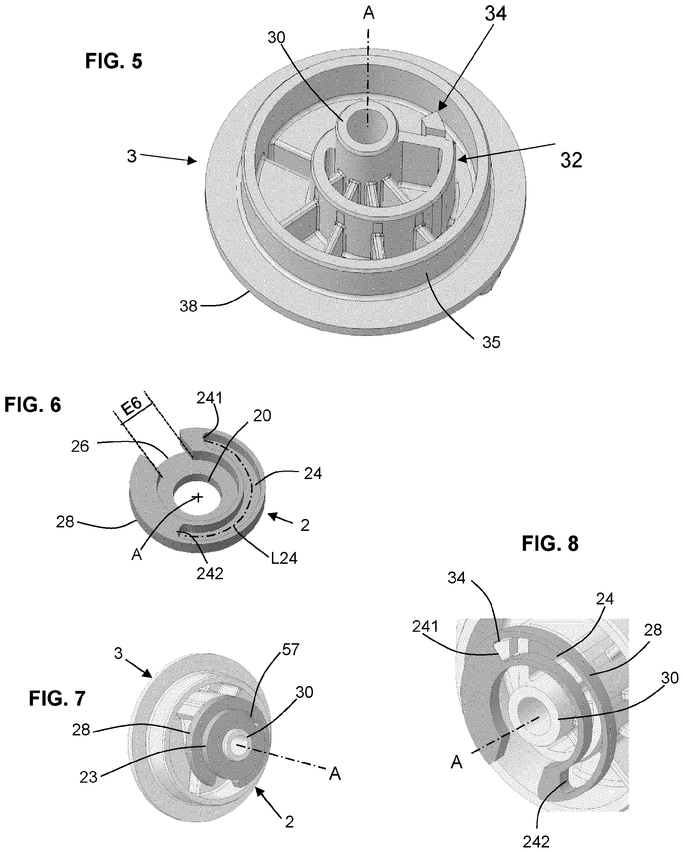

**FIG. 3** illustrates an exploded perspective view of a distribution device 10 according to the present disclosure. **FIG. 4** - **FIG. 8** provide additional views including details.

The distribution device **10** comprises a body **1.** The body is made of molded plastics.

The distribution device **10** comprises a selector hub **2,** pivotally mounted within the body **1** about a main axis denoted **A.** The selector hub **2** comprises a cylindrical outer journal bearing interface **23,** which is received in a counterpart inner bearing **13** of the body **1.** The selector hub **2** comprises an outer rim **28,** designed to push outwardly the pinch elements **4.**

The selector hub **2** comprises a cylindrical inner journal bearing **20** to receive a shaft portion of the control member discussed later.

The selector hub **2** comprises an arcuate groove **24.** The curved centerline **L24** of the arcuate groove **24** follows an arc of circle centered on the device axis **A.**

The body comprises a plurality of passages **12,** each passage being configured to accommodate/house one flexible tube **5** to be selectively pinched by the device. In one embodiment, each passage is formed with the insertion slot **95** as depicted in **FIG. 2A** and

**FIG. 2B****.** However, it is not excluded to have a passage deprived of an insertion slot as illustrated at **FIG. 11****.**

Moreover, as shown at the various figures, the cross section of the passage **12** can be circular or can be square. Other shapes of cross section are note excluded though.

The body **1** comprises a plurality of guide slots **21** that are discussed later. Each guide slot **21** comprises lateral walls **22** that are made integral with the body **1.** Each guide slot provides a slide arrangement for the pinch element housed therein.

The distribution device **10** comprises a set of pinch elements **4,** each pinch element being involved in one pinch valve portion.

Each pinch element is movable and is guided in a guide slot **21** in a radial direction about the main axis **A.** As visible on figure 20, the guide slot comprises two spaced apart, parallel walls **211,212.** The width of the pinch element 4 is slightly lower than the distance separating the two walls 211,212.

Each pinch element exhibits a front end **41** that can extend within the passage. The front end **41** is movable into the passage and back, whereby each pinch element **4** is configured to selectively pinch or release one tube. Each pinch element exhibits a back end **44** configured to be a contact either with the outer rim **28** of the selector hub **2,** or in contact with the cam portion **32** of the control member, or in contact with both. Each pinch element **4** comprises side walls **46** adapted to slide within the walls **22** of the guide slot **21.** The outer rim **28** is interrupted at least at one location where it does not prevent the pinch element to retract toward the main axis **A.** At this place, a recess **26** is formed.

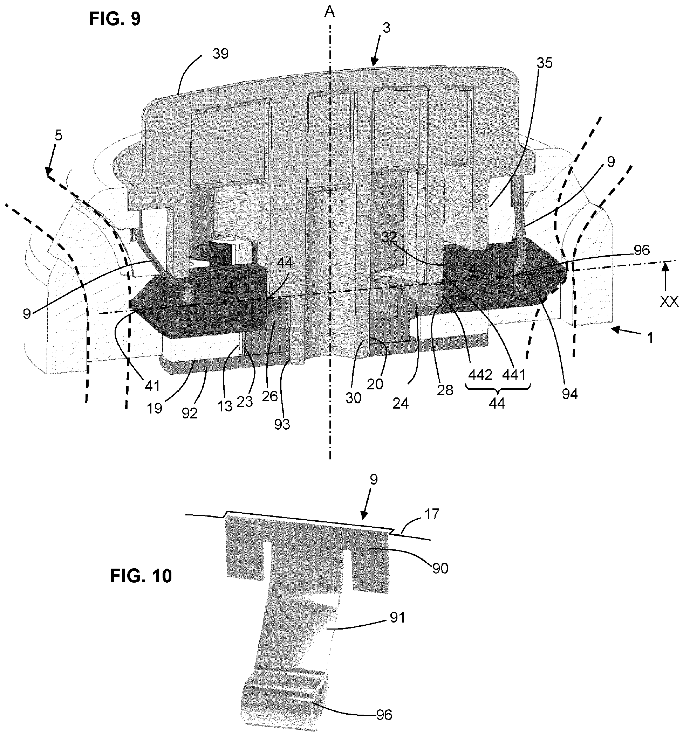

As shown at **FIG. 9****,** left side, the flexible tube **5** is not pinched, whereas by contrast at right side, the pinch element **4** is pushed outwards and pinches the flexible tube **5.**

In the illustrated example, they are provided eight guide slots **21,** eight pinch elements **4,** eight passages **12.** Each set of (passage + pinch element + guide slot + tube therein) forms an individual pinch valve. Each individual pinch valve is completely separated from another individual pinch valve: this ensures avoiding any cross contamination between the individual pinch valves.

However, the number **M** of passages, guide slots and pinch elements can be less, for example from 2 to 7, or can be more, for example 10 or 12, without excluding any other value.

Turning back to the example with eight individual pinch valves, i.e., eight (passage + pinch element + guide slot), they are evenly distributed along the circumference, namely one pinch valve every 45°.

For another implementation with 6 pinch valves, they would be distributed every 60°. For another implementation with 10 pinch valves, they would be distributed every 36°. A non-even distribution along the circumference is not excluded.

The distribution device **10** comprises a control member **3.** The control member **3** is pivotally mounted within the body about the axis **A.**

The control member 3 comprises a shaft **30** extending along the main axis into the cylindrical inner journal bearing **20** of the selector hub **2.**

The control member **3** comprises an outer rim **35** received in a cylindrical bearing **15** of the body. The control member **3** comprises an upper outer border **38** pivotally mounted in an upper inner border **16** of the body **1.**

The control member comprises a cam portion **32** configured to cause or allow a radially oriented movement of the pinch elements when the control member is rotated around the main axis. In the illustrated example, the cam portion **32** pushes outwards the pinch elements. In alternate configurations not shown, the cam portion **32** could pull the pinch elements in the inwards direction.

The control member **3** comprises a driving pin **34** extending along a direction parallel to main axis **A.** The driving pin **34** is received in the arcuate groove **24** of the selector hub 2. The driving pin **34** is free to move along the arcuate groove **24.** The length, i.e., angular aperture of the arcuate groove defines the length of the free range. The angular aperture corresponding to said free play is name here "first angular range".

The arcuate groove is delimited at its both ends by the first abutment **241** and a second abutment **242.** In the clockwise direction **CW,** the driving pin 34 abuts against the first abutment **241,** whereas in the counterclockwise direction **CC,** the driving pin **34** abuts against the second abutment **242.**

The distribution device **10** comprises a closing washer **92,** arranged at the bottom section **19** of the body, which is generally opposite the upper surface of the control member **3** in the overall assembly. The washer **92** is adjacent to a bottom plane **19** of the body **1.** In the illustrated example, the shaft **30** of the control member is received in a center bore **93** of the washer **92.**

It should be understood that, in the illustrated example, the control member 3 and the washer **92** sandwich the selector hub **2** within the body. Note that corresponding fastening means are not shown at the figures.

Turning now to **FIG. 4****,** there is provided a one-way rotational system to allow the selector hub to be rotated in the counterclockwise direction and to prevent the selector hub to be rotated in the clockwise direction.

More precisely, in the example, there is provided a leaf spring **58** arranged in a slot 57. The leaf spring slot **57** extends substantially in a tangential direction.

The free end **58a** of the spring abuts against shoulders **18** provided at the outer journal bearing interface **23** of the body. When the selector hub rotates in the counterclockwise direction the leaf spring **58** bends and passes over the shoulder **18.** By contrast, when the selector hub rotates in the clockwise direction, free end **58a** of the spring abuts against shoulders **18,** thereby preventing further national movement.

It should be understood that the driving pin could be arranged on the selector hub **2** and the arcuate groove can be arranged on the control member, which corresponds to an inverted configuration about the one illustrated above. Further, any other driving mechanism with a large angular free play can also be considered.

Therefore, the present disclosure encompasses any mechanical coupling arrangement, wherein the control member is coupled in rotation with the selector hub for allowing a free relative rotation along a first angular range and a rotationally rigid engagement beyond said first angular range. We note that an endless rotation of the selector hub in the clockwise direction CW is possible.

In the illustrated example, the recess **26** provided in the outer rim **28** covers an angular range **E6** for only one pinch element. Except at the location of this recess, the outer ring **28** maintains all the pinch elements **4** in the outwardly pushed position. Only the pinch element located opposite the recess **26** can be moved inwards in the radial direction. In the present case N=1.

As apparent from **FIG. 9** right side, the lower back end **442** of the pinch element is interfering with the outer rim **2** of the selector hub **2.** The upper back end **441** of the pinch element is interfering with the cam portion **32** of the control member **3.**

Turning now to **FIG. 16****,** where the viewpoint to from top area, the clockwise direction is denoted **CW,** whereas the counterclockwise direction is denoted **CC.** Generically, we can use the terms of `first rotational direction' and 'second rotational direction', with no reference to clock.

In the process depicted at **FIG. 16A- 16D****,** the body **1** remains stationary, the guide slots also remain stationary.

The state shown at **FIG. 16A** shows one pinch element retracted (ref **401**), i.e., one tube released (pinch valve open). The other pinch elements **402, 403** are maintained in outwards position by the outer rim **28** of the selector hub. Note that in **FIG. 16A** the control member is not shown.

At **FIG. 16B****,** the selector hub **2** is in the same position as per **FIG. 16A****,** and the control member **3** has been rotated in counterclockwise direction by 180°. During said rotation, the cam member has gradually pushed outwards the pinch element **401.** Consequently, the flexible tube located in front of this pinch element **401** is squeezed and the passage is closed.

Angular length **of L24** is consistent with the angular length of the cam ramp. It can be any value in the range 45°-315°. In one exemplary configuration, the angular length of the cam ramp can be comprised between 90° and 270°. In the illustrated example the angular length of the cam ramp is approximately 180°. Generally, we may choose the first angular range comprised between 100° and 180°.

In **FIG. 16C** the control element **3** is further rotated in counterclockwise direction **CC** together with the selector hub. More precisely the driving pin **34** abuts on the end abutment **241** of the arcuate groove **24** and therefore the driving pin **34** drives the selector hub **2** in counterclockwise direction concurrently with the control member **3.**

In the illustrated example, the rotation in counterclockwise direction exhibits an angular range corresponding to two pinch elements angular pitch. Here with **8** passages regularly spaced along the circumference, the angular range corresponding to two pinch elements is 90°. Otherwise, the centerline **L0** of the recess is rotated 90° in the counterclockwise direction. All the pinch elements remain in the outwards position, pinch element reference **403** is pushed by the cam portion **32** whereas the other pinch elements are pushed in the outwards position by the outer rim 28 of the selector 2.

In **FIG. 16D** the control element has rotated in clockwise direction **CW** by 180°. However, the pinch element reference **403** is allowed to move inwardly toward the axis. Thereby, the passages in front of pinch elements **403** is released. There is no possibility or risk of moving the selector hub in the counterclockwise direction thanks to the one-way rotation system described above.

As apparent from **FIG. 1** and **FIG. 9****,** the top side of the control member **3** comprises a knob **39** to allow easy handling by a user hand. Also, according to an advantageous option, the knob can be a straight upstanding protrusion. Such top protrusion can be manually actuated, i.e., by fingers of a user.

In the illustrated example, the knob is aligned with the selection of the passage to be released, according to the general configuration shown at **FIG. 1A****.** This provides an intuitive positioning indication for the user.

### Biasing spring

As illustrated at **FIG. 9** and **FIG. 10**, there may be provided, in addition to the features already disclosed above, biasing springs **9.** There is provided one biasing spring for each pinch element.

Each biasing spring comprises a base portion **90,** a resilient intermediate portion **91,** and a pusher portion **96.** The base portion **90** is fixedly secured to the body, for example by means of a recess indentation **17.** The base portion **90** is held in place in the indentation by the control member 3 which closes inwardly the indentation.

The pusher portion **96** is received in a recess **94** provided in the corresponding pinch element. The pusher portion **96** has preferably a convex shape. As the resilient intermediate portion flexes, the pusher portion **96** substantially moves inwardly or outwardly.

Each biasing spring **9** can be made from a metallic blank or can be made as a molded plastic part. Each biasing spring **9** pushes inwards a pinch element.

Depending on the nature and resilience of the flexible tube, the biasing spring **9** exerts a strong and predictable force to reliably release the flexible tube when the state open each valve is required. In this case, the tube does not need to push back the pinch element. The resilience of the tube only restores its rest geometry.

The rest position is depicted at **FIG. 9** left side and the deflected position is depicted at **FIG. 9** right side.

### Motor assisted rotation

As depicted in **FIG. 11****,** in addition to manual operation of the control member there may be provided a motorized rotation of the control member. They provided an electric motor **6** comprising a rotor rigid in rotation with the shaft **30** of the control member. The electric motor **6** can be controlled in both rotational directions.

The distribution device can be actuated either manually or through the electric motor **6.** In the exemplified configuration, the motor is controlled via an electric wire **61.**

There may be provided an angular encoder, or an angular reference 0 position to contribute to a reliable control from the applicant motor.

The other components provided in the distribution device are not described again since they are similar or identical to the components already disclosed for the first embodiment.

### Larger Recess

**FIG. 12** and **FIG. 13** illustrate a variant where the recess **260** provided in the outer rim **28** extends over an angular range encompassing two pinch elements. The angular range **E62** is here 90°

Pinch elements referenced **408, 409** can be moved inwards inside the recess **260.** The other pinch elements are kept in the outwards position by the outer rim **28.** In the present case N=2.

In this variant, two pinch valves can be open at the same time, depending on the ramp shape of the control member.

The other components provided in the distribution device are not described again since they are similar or identical to the components already disclosed for the first embodiment.

### Sensing Elements

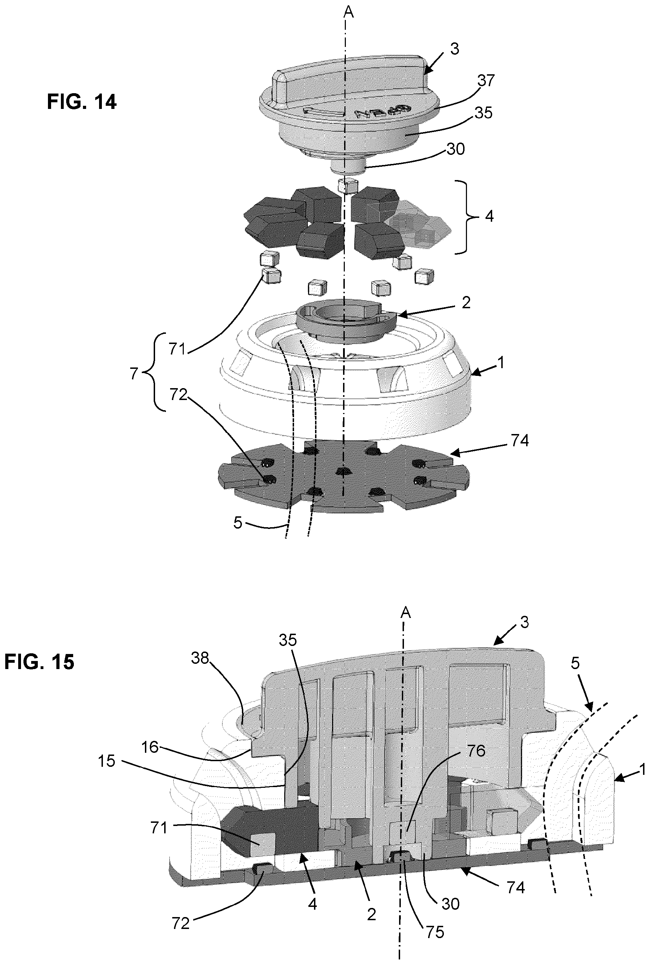

As illustrated in **FIG. 14** and **FIG. 15**, there may be provided additionally sensing means 7 to sense the actual position of each pinch element **4.**

For each pinch element **4,** a magnet **71** is lodged in a bottom area of the pinch element **4.** A Hall-effect sensor **72** is arranged opposite the magnet when pinch element is in the outwards position. Therefore, the position which is detected positively easy outwards position corresponding to a close state of the corresponding pinch valve.

There is provided a printed circuit board **74** for supporting the Hall-effect sensors **72.**

Said sensing means **7** allows a close loop control in case the distribution is controlled by a control unit via the electric motor **6.**

The is provided additionally a center Hall-effect sensor **75** and a center magnet **76.** The purpose is to be able to check the proper assembly of the distribution device.

The other components provided in the distribution device are not described again since they are similar or identical to the components already disclosed for the first embodiment.

### Stack of Devices

As illustrated at **FIG. 17**, it is considered an assembly of two or more distribution devices as described above, with a common center shaft **31.** The center shaft can be made integral with the local shafts **30** of each distribution device. Alternately, the center shaft can be distinct but rigid in rotation with the local shafts **30** of each distribution device.

In the exemplified variant, an electric motor **6** is provided to drive the center shaft **31.** However, it is possible to have a manual control of the shaft when the top distribution device comprises a control knob, as the one depicted earlier.

Several devices are stacked one above the other. The shafts of the devices are rigid in rotation with one another. The devices may not be adjacent to one another, there may a gap provided between two devices. This in-between gap can be useful to provide enough room to let tubes go in and out of the respective passages **12.**

The other components provided in the distribution device are not described again since they are similar or identical to the components already disclosed for the first embodiment.

### Collective Control

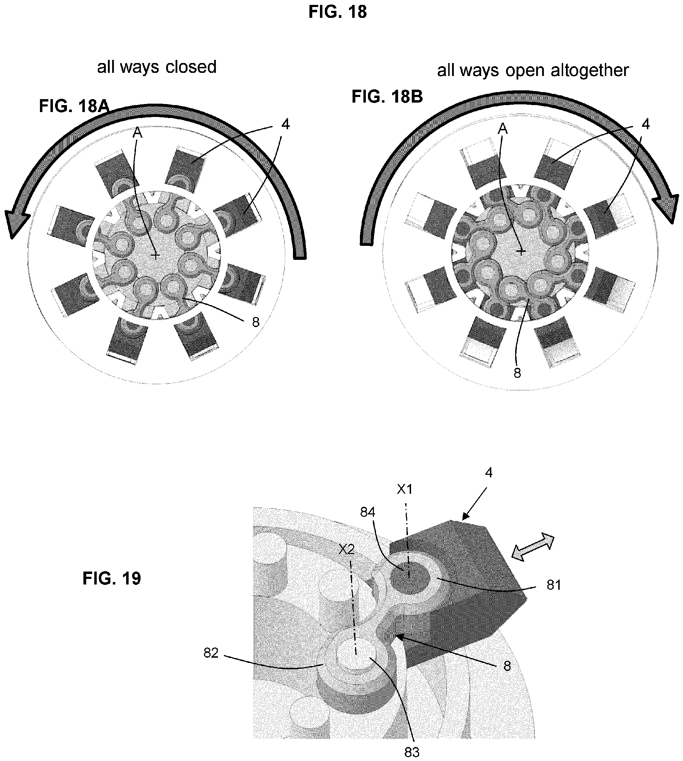

As illustrated at **FIG. 18** and **FIG. 19****,** in this further embodiment, all the pinch elements **4** are controlled the same way by rotation of the control member **3.**

In this embodiment, the selector hub is not necessary and can be omitted. Only the control member **3** rotates around axis **A** within the body **1.** The pinch elements are received slidingly in guide slots as described for the first embodiment.

The mechanical interaction between the control member **3** and the pinch elements **4** is formed by a set of connecting rods **8.** Each connecting rod **8** comprises a first end **81** coupled to a back end of one pinch element. Each connecting rod **8** comprises and a second end **82** coupled to an attachment portion of the control member.

There is provided a first pivot pin **84** at the pinch element. At this place, the first pivot pin and the connecting eyelet form a first joint axis **X1**.

There is provided a second pivot pin **83** at the control member **3.** At this place, the second pivot pin and the other connecting eyelet form a second joint axis **X2.**

The control member **3** can rotate around axis **A** within a predefined angular range. In the example with M=8, the predefined angular range is about 40°.

In one embodiment, the predefined angular range is available between a first angular stop and a second angular stop.

When the control member abuts against the first angular stop, the pinch elements are completely withdrawn from the passages, and shown at **FIG. 18B****.**

When the control member abuts against the second angular stop, the pinch elements are caused to pinch the tubes held in the passages to obstruct any fluid flow in the tubes and shown at **FIG. 18A****.**

Generally speaking, the predefined angular range can be comprised between 25° and 50°.

When the control member abuts against the second angular stop, the main axis **A,** the first joint axis and the second joint axis are substantially aligned for each couple of rod and pinch element.

In this embodiment, the control member **3** can be rotated manually or via an output shaft controlled by a motorized actuator **6.**

Generally speaking, the control member allows a mechanical interaction between the control member and the pinch elements so as to cause a radially oriented movement of all the pinch elements altogether when the control member is rotated around the axis.

### Miscellaneous

It should be understood that the features set forth for any one embodiment can be combined with features set forth for in any other embodiments described herein.

Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein.

It is to be understood that the present disclosure is not limited to the aspects described above and illustrated in the drawings; rather, the skilled person will recognize that many changes and modifications may be made within the scope of the present disclosure and appended claims. In the drawings and specification, there have been disclosed aspects for purposes of illustration only and not for purposes of limitation, the scope of the inventive concepts being set forth in the following claims.

## Claims

1. A device for holding a set of M flexible tubes and for selectively pinching or releasing all or a subset of N tubes among the set of M flexible tubes, the device comprising:
a body **(1),** having M passages **(12),** each passage being configured to accommodate one tube, the body having a main axis **(A);**
a set of pinch elements **(4),** each pinch element being guided in a guide slot and radially movable in the passage about the main axis, each pinch element being configured to selectively pinch or release one tube;
a selector hub **(2),** pivotally mounted within the body about the main axis, wherein an angular position of the selector hub around the main axis causes to select the subset of N tubes, by selecting a corresponding subset of pinch elements to be allowed to move;
a control member **(3)** pivotally mounted within the body about the main axis, the control member having a cam portion **(32)** configured to cause or allow a radially oriented movement of some the pinch elements when the control member is rotated around the main axis; and
a mechanical coupling arrangement **(24,34),** wherein the control member is coupled in rotation with the selector hub for allowing a free relative rotation along a first angular range and a rotationally rigid engagement beyond said first angular range.

2. The device according to claim 1, wherein a number M-N tubes are normally pinched by the pinch elements and the selector hub, and the device is configured to release only one or two selectable tubes, i.e. N =1 or N=2.

3. The device according to any of claims 1 to 2, wherein the selector hub **(2)** comprises a latch member **(28)** configured to prevent a rotation of the selector hub along a first rotational direction and to allow a rotation of the selector hub along a second rotational direction, and wherein when the control member is rotated along a first rotational direction the cam portion **(32)** causes a radial movement of some the pinch elements.

4. The device according to any of claims 1 to 3, wherein the control member comprises a top protrusion **(39)** that can be manually actuated, i.e., by fingers of a user.

5. The device according to any of claims 1 to 3, wherein the control member is rotated via an output shaft controlled by a motorized actuator **(6).**

6. The device according to any of claims 1 to 5, wherein the first angular range is comprised between 100° and 180°.

7. The device according to any of claims 1 to 6, wherein the mechanical coupling arrangement is such that the control member comprises a pin **(34)** engaged in an arcuate groove **(24)** arranged in the selector hub, the available angular stroke of the pin within the arcuate groove defining the first angular range.

8. The device according to any of claims 1 to 7, wherein the passages are disposed in a circle and passages are regularly spaced circumferentially.

9. The device according to any of claims 1 to 8, wherein **M** is at least 4 and the device has at least four passages and accommodate at least four tubes.

10. The device according to any of claims 1 to 9, wherein the selector hub comprises an outer rim **(28)** interrupted by a recess **(26).**

11. The device according to any of claims 1 to 10, wherein each passage comprises an insertion slot **(95).**

12. The device according to any of claims 1 to 11, wherein there is provided sensing means (7) to sense the radial position of each pinch element.

13. The device according to any of claims 1 to 12, wherein each pinch element comprises a magnet **(71)** and there is provided a Hall-effect sensor **(72)** located opposite the magnet when pinch element pushed radially outwards, i.e., when the tube is pinched.

14. The device according to any of claims 1 to 13, wherein there is provided biasing members **(9)** to push the pinch elements radially inwards.

15. An assembly comprising at least two devices according to any preceding claim and a control shaft controlled by motorized actuator.
